# FASCICULE DE BREVET EUROPEEN

(11) **EP 3 302 577 B1**
(45) Date de publication et mention de la délivrance du brevet: **26.06.2019**
(21) Numéro de dépôt: 16725120.6
(22) Date de dépôt: 24.05.2016
(51) Int. Cl.: A61K 49/00, C07K 7/64

(54) **TRACEUR FLUORESCENT CIBLANT MULTIVALENT OPTIMISE**
OPTIMIERTE MULTIVALENTE FLUORESZIERENDE TRACER ZUM TARGETING
OPTIMISED MULTIVALENT TARGETING FLUORESCENT TRACER

(30) Priorité: 25.05.2015 FR 1554669
(43) Date de publication de la demande: 11.04.2018
(73) Titulaire: Fluoptics, 38040 Grenoble (FR)
(72) Inventeur: GAYET, Pascal, 38040 Grenoble (FR)
(74) Mandataire: Esselin, Sophie
(86) Numéro de dépôt international: PCT/EP2016/061715
(87) Numéro de publication internationale: WO 2016/189007

(56) Documents cités:
- US-A1- 2014 271 482
- Elisabeth Garanger: "Conception, Synthèse et Caractérisation de Nouveaux Systèmes de Guidage et de Vectorisation pour la Cancérologie", Thèse - Université Joseph Fourier - Grenoble I, 26 juillet 2005 (2005-07-26), XP055262866, Extrait de l'Internet: URL:https://tel.archives-ouvertes.fr/tel-0 0009834/document [extrait le 2016-04-05]

## Description

La présente invention concerne des composés tels que des sondes de fluorescence dans le domaine du proche infrarouge. Ces composés comprennent des molécules de ciblage de tissus ou d'organes cibles, un fluorophore et un support sur lequel sont fixés les molécules de ciblage et le fluorophore.

### ARRIERE PLAN TECHNOLOGIQUE

L'imagerie de fluorescence est en plein essor dans de nombreuses applications chirurgicales. Plusieurs dispositifs médicaux sont sur le marché permettant la détection et la visualisation d'un des seuls fluorophores disposant d'une autorisation de mise sur le marché : le vert d'indocyanine. Toutefois, ce fluorophore est une molécule non spécifique de tissus ou d'organes cibles ce qui restreint son champ d'application.

L'imagerie médicale est une technique prometteuse dans les interventions chirurgicales. Elle peut, par exemple, servir à guider le chirurgien lors de son intervention. Cette technique repose sur l'administration au patient d'un traceur fluorescent. En l'espèce, il s'agit d'un traceur fluorescent ciblant des tissus ou organes nécessitant l'intervention chirurgicale.

Le principe repose sur un éclairement par une source lumineuse d'un traceur fluorescent, préalablement administré au patient, spécifique de tissus ou d'organes cibles. L'éclairement a pour effet d'exciter ledit traceur fluorescent qui émet alors à son tour un rayonnement à une longueur d'onde donnée. Les principales applications sont dans le domaine du proche infrarouge entre 700 nm et 1000 nm. En effet, cette fenêtre optique correspond au domaine de longueurs d'ondes dans lequel les tissus biologiques absorbent le moins.

L'intérêt des traceurs fluorescents est qu'ils permettent d'associer des propriétés d'au moins deux classes de molécules telles que des propriétés de fluorescence d'une part, et, des propriétés de ciblage de tissus ou d'organes spécifiques d'autre part.

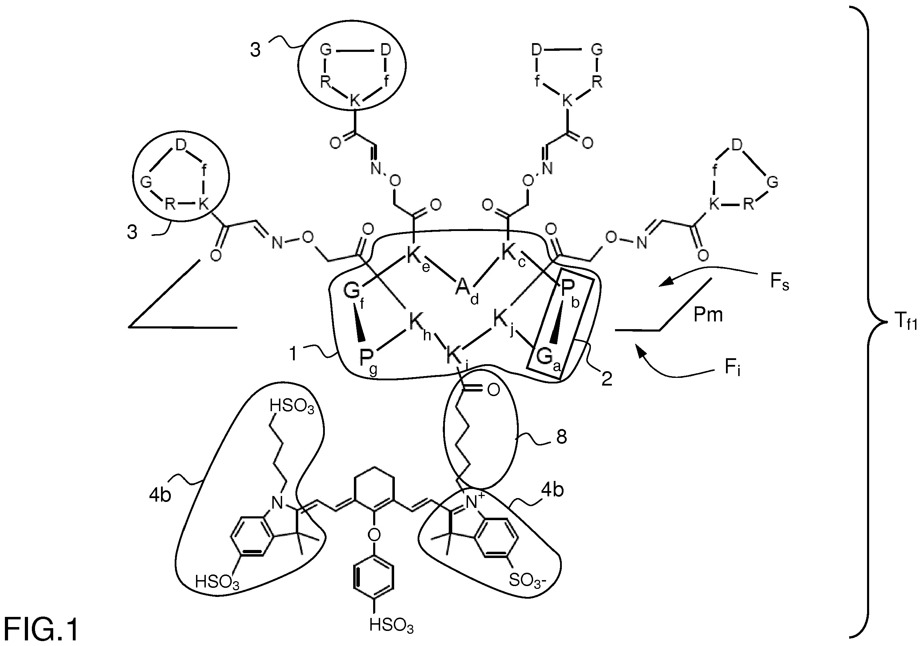

Dans cette optique, il a été proposé un traceur fluorescent dans le domaine du proche infrarouge spécifique de l'intégrine αᵥβ₃, représenté sur la figure 1.

Le traceur fluorescent **Tf1** comprend un châssis moléculaire **1**, des molécules de ciblage **3** et un fluorophore **4**.

Le châssis moléculaire 1 est un décapeptide cyclique RAFT, acronyme de « Regioselectively Adressable Functionnalized Templates », en langue anglaise, ou châssis fonctionnalisable régio-sélectivement.

Il s'agit d'un décapeptide cyclique comprenant la séquence de résidus d'acides aminés : -Glycineₐ-Proline_{b}-Lysine_{c}-Alanine_{d}-Lysineₑ-Glycine_{f}-Proline_{g}-Lysineₕ-Lysineᵢ-Lysineⱼ- **[-Gₐ-P_{b}-K_{c}-A_{d}-Kₑ-G_{f}-P_{g}-Kₕ-Kᵢ-Kⱼ]**, les enchaînements des résidus acides aminés Glycine et Proline **G_{a;f}**; **P_{b;g}** formant des coudes **2** de sorte que la configuration du châssis moléculaire **1** présente un plan moyen **Pm** définissant une face dite supérieure **Fₛ** comprenant quatre résidus Lysine **K_{c}**, **Kₑ**, **Kₕ** et **Kⱼ** du décapeptide cyclique et une face dite inférieure **Fᵢ**, opposée à la face supérieure **Fₛ** comprenant le résidu Lysine **Kᵢ**.

On entend par plan moyen **Pm** le plan pour lequel la somme des distances entre le plan moyen **Pm** et les résidus d'acides aminés est minimale.

Le décapeptide cyclique RAFT ainsi réalisé permet une présentation de molécules de ciblage multimérique et peut être associé de manière contrôlée à deux domaines fonctionnels indépendants : un domaine dédié au ciblage de zones d'intérêt, telles que des zones exprimant des intégrines, et un domaine pour la détection.

Les molécules de ciblage **3** sont des pentapeptides cycliques comprenant la séquence de résidus d'acides aminés **-RGD-.**

De nombreuses intégrines interagissent avec leurs substrats protéiques via la séquence de résidus d'acides aminés **-RGD-** acronyme de « Arginine-Glycine-Acide Aspartique ». Cette séquence RGD est un motif commun présent sur la plupart des protéines de la matrice extracellulaire. Les molécules pentapeptidiques cycliques **3** sont couplées aux quatre résidus d'acides aminés Lysine **K_{c}**, **Kₑ**, **Kₕ** et **Kⱼ** de la face dite supérieure **Fₛ** du châssis moléculaire 1 via des liaisons oximes.

Le fluorophore **4** de la famille des cyanines fluoresce dans le domaine de longueurs d'onde compris entre 700 et 900 nm. Notons que le fluorophore **4** est ici l'IRDye800 (marque déposée) mais peut également être de la cyanine 5 (marque déposée), l'Alexa fluor 700 (marque déposée) ou un fluorophore développé spécifiquement à 700 nm noté BM 105 (marque déposée).

Le fluorophore **4** est relié au résidu Lysine **Kᵢ** de la face dite inférieure **Fᵢ** du châssis moléculaire **1** en formant une liaison amide via un groupement aliphatique d'un des groupements aromatiques indol **4b** du fluorophore **4.**

Le traceur fluorescent **Tf1** réalisé selon l'art connu présente un maximum d'absorption à 781 nm et un maximum d'émission à 801 nm, il est particulièrement bien adapté au dispositif d'imagerie Fluobeam (marque déposée) de Fluoptics.

Le procédé de réalisation du traceur fluorescent **Tf1** selon l'état de la technique comprend :
- une première étape de synthèse du châssis RAFT 1 comprenant :
   ∘ une sous-étape de synthèse d'un décapeptide linéaire [-K(boc)-K(Alloc)-K(Boc)- P-G-K(Boc)- A-K(Boc)-P-G] sur résine,
   ∘ une sous-étape de cyclisation du décapeptide en solution,
   ∘ une sous-étape de déprotection des K(Boc),
   ∘ une sous-étape de greffage d'un précurseur d'oxyamine protégé,
   ∘ une sous-étape de déprotection des K(Alloc),
- une deuxième étape de synthèse des molécules de ciblage **3**, dans le cas présent, les pentapeptides cycliques présentant l'enchaînement de résidus d'acides aminés : Acide aspartique, phénylalanine, Lysine, Arginine et Glycine [-D(tBu)-F-K(Alloc)-R(Pbf)-G-] **3**,
- une troisième étape de couplage entre le châssis RAFT **1** et des molécules de ciblage **3** comprenant :
   ∘ une sous-étape de cyclisation des pentapeptides en solution,
   ∘ une sous-étape de déprotection de K(Alloc),
   ∘ une sous-étape de greffage de S protégé,
   ∘ une sous-étape de déprotection de D, de S et de R, et
   ∘ une sous-étape d'oxydation de S,
- une quatrième étape d'activation du fluorophore, et
- une cinquième étape de couplage, ultérieure aux troisième et quatrième étapes, entre le châssis RAFT comprenant la molécule de ciblage **3** et le fluorophore **4**.

En pratique, le fluorophore **4** est commercialisé sous la forme activée ce qui le rend particulière instable et réactif notamment vis-à-vis des amines.

Les rendements de ce procédé sont relativement faibles et notamment le rendement de couplage entre le châssis RAFT **1** comprenant les molécules de ciblage 3 et le fluorophore **4**. En effet, pour obtenir 15g de traceur fluorescent **Tf1** tel que celui décrit figure 1, 410 g de molécule de ciblage **3**, 75g de châssis RAFT **1** et 7g de fluorophore **4** sont nécessaires.

Les coûts globaux de réalisation d'un traceur fluorescent **Tf1** selon l'art connu sont très élevés en raison d'une part du coût d'achat du fluorophore **4**, et, d'autre part du procédé de synthèse qui génère des pertes de matières premières importantes. Elisabeth Garanger dans "Conception, Synthèse et Caractérisation de Nouveaux Systèmes de Guidage et de Vectorisation pour la Cancérologie", Thèse - Université Joseph Fourier - Grenoble I, 26 juillet 2005 divulgue un traceur fluorescent comprenant le fluorophore Cy5, le châssis RAFT et quatre molécules de ciblage RGDfK.

Un but de la présente invention est de proposer une alternative au traceur fluorescent proposé selon l'art antérieur, et surmontant le problème précité. Aussi, le traceur fluorescent selon l'invention est développé spécifiquement pour permettre une excellente compatibilité avec un dispositif d'imagerie en proche infrarouge tel que le dispositif Fluobeam (marque déposée) de Fluoptics et présente un coût inférieur à celui des traceurs proposés selon l'état de la technique. En effet, le dispositif Fluobeam (marque déposée) comprend un laser d'excitation à 750 nm permettant l'obtention du maximum d'intensité de fluorescence du traceur fluorescent selon l'invention.

### RESUME DE L'INVENTION

Selon un aspect de l'invention, il est proposé un traceur fluorescent comprenant :
- un décapeptide cyclique constitué de l'enchaînement des dix résidus d'acides aminés : -Glycine-Proline-Lysine-Alanine-Lysine-Glycine-Proline-Lysine-Lysine-Lysine configuré de sorte à définir un plan moyen définissant une première face supérieure présentant quatre résidus d'acides aminés Lysine et une deuxième face inférieure présentant un résidu d'acide aminé Lysine,
- quatre molécules de ciblage identiques, les molécules de ciblage étant des pentapeptides cycliques constitués de l'enchaînement de résidus d'acides aminés : Arginine - Glycine - Acide aspartique - Phénylalanine - Lysine, chacune des molécules de ciblage étant fixée sur un résidu d'acide aminé Lysine différent de la première face supérieure via une liaison oxime, et
- un fluorophore sel interne (sel trisodique) hydroxyde de 3,3-Dimethyl-2-[2-[2-chloro-3-[2-[1,3-dihydro-3,3-dimethyl-5-sulfo-1-(4-sulfobutyl)-2H-indol-2-ylidene]-ethylidene]-1-cyclohexen-1-yl]-ethenyl]-5-sulfo-1-(4-sulfobutyl)-3H-indolium présentant un enchaînement de doubles liaisons avec un carbone central, le fluorophore étant fixé sur la deuxième face inférieure du plan moyen via un bras espaceur reliant le carbone central de l'enchaînement de doubles liaisons du fluorophore, via une liaison éther, et, le résidu d'acide aminé Lysine de la face inférieure du décapeptide via une liaison amide, le bras espaceur étant l'acide 5-(4-hydroxyphenyl)pentanoïque..

Le traceur ainsi proposé est bien adapté au dispositif d'imagerie Fluobeam (marque déposée) de Fluoptics et possède un rendement quantique six fois plus grand que celui obtenu avec le vert d'indocyanine actuellement utilisé en clinique.

Selon un autre aspect de l'invention, il est proposé un procédé de synthèse d'un traceur fluorescent, le traceur fluorescent comprenant :
- un décapeptide cyclique constitué de l'enchaînement des dix résidus d'acides aminés : -Glycine-Proline-Lysine-Alanine-Lysine-Glycine-Proline-Lysine-Lysine-Lysine- configuré de sorte à définir un plan moyen définissant une première face supérieure présentant quatre résidus d'acide aminé Lysine et une deuxième face inférieure présentant un résidu d'acide aminé Lysine,
- quatre molécules de ciblage identiques, les molécules de ciblage étant des pentapeptides cycliques constitués de l'enchaînement de résidus d'acide aminé : Arginine - Glycine - Acide aspartique - Phénylalanine - Lysine, chacune des molécules de ciblage étant fixée sur un résidu d'acide aminé Lysine différent de la première face supérieure via une liaison oxime, et,
- un fluorophore sel interne (sel trisodique) hydroxyde de 3,3-Dimethyl-2-[2-[2-chloro-3-[2-[1,3-dihydro-3,3-dimethyl-5-sulfo-1-(4-sulfobutyl)-2H-indol-2-ylidene]-ethylidene]-1-cyclohexen-1-yl]-ethenyl]-5-sulfo-1-(4-sulfobutyl)-3H-indolium, présentant un enchaînement de doubles liaisons avec un carbone central, le fluorophore S0456 étant fixé sur la deuxième face inférieure du plan moyen via un bras espaceur reliant le carbone central de l'enchaînement de doubles liaisons du fluorophore via une liaison éther et le résidu d'acide aminé Lysine du décapeptide via une liaison amide, le bras espaceur étant un acide 5-(4-hydroxyphenyl)pentanoïque,
le procédé comprenant une étape de fixation du fluorophore sur le décapeptide via le bras espaceur préalablement à une étape de fixation des molécules de ciblage permettant ainsi de supprimer l'étape d'activation du fluorophore.

### LISTE DES FIGURES

L'invention sera mieux comprise et d'autres avantages apparaîtront à la lecture de la description qui va suivre et, grâce aux figures annexées parmi lesquelles :
- la figure 1, déjà décrite, représente un traceur fluorescent selon l'art connu,
- la figure 2 représente le traceur fluorescent développé pour le dispositif d'imagerie Fluobeam (marque déposée) de Fluoptics, selon l'invention,
- la figure 3 est une superposition des spectres d'absorption des traceurs fluorescents selon l'art connu et selon l'invention,
- la figure 4 est une superposition des spectres d'émission des traceurs fluorescents selon l'art connu et selon l'invention,
- la figure 5a et 5b mettent en évidence la distribution tissulaire des traceurs fluorescents selon l'art connu et selon l'invention,
- la figure 6 représente les réactions de couplage entre le bras espaceur et le fluorophore selon l'invention,
- les figures 7a et 7b représentent les réactions de synthèse du châssis RAFT, et de couplage entre le fluorophore muni du bras espaceur et le châssis RAFT, selon l'invention,
- la figure 8 représente les réactions de synthèse des molécules de ciblage selon l'invention,
- la figure 9 représente la réaction de couplage entre le châssis RAFT fluorescent et les molécules de ciblage via une liaison amide selon l'invention.

### DESCRIPTION DETAILLEE

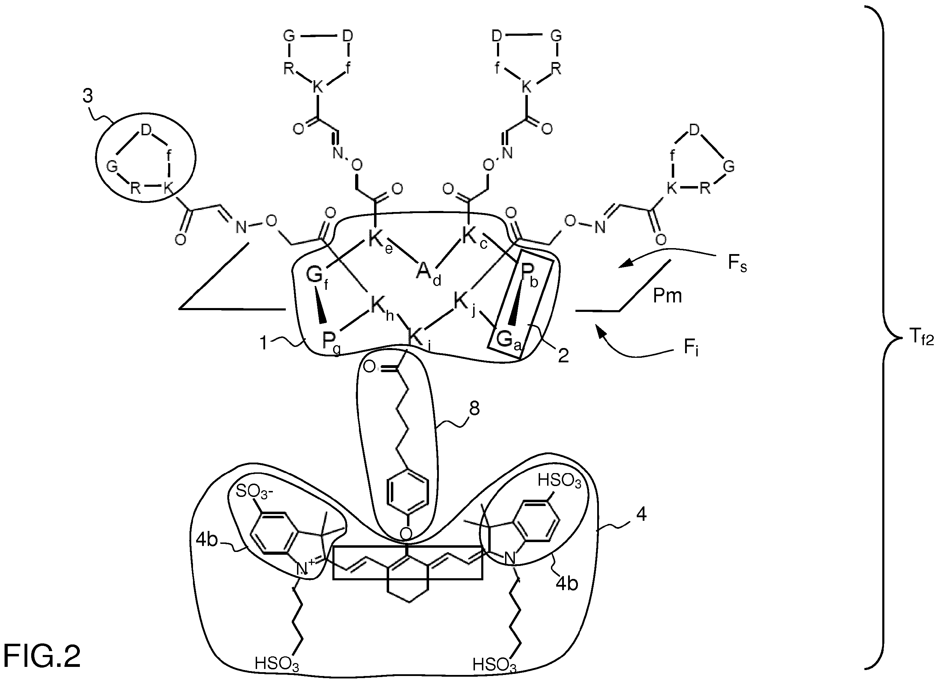

La **figure 2** représente le traceur fluorescent Tf selon l'invention.

Comme le traceur **Tf1** de l'état de la technique décrit à la figure 1, le traceur **Tf2** selon l'invention comprend un support moléculaire **1** sur lequel sont fixés des molécules de ciblage **3** et le fluorophore **4**.

Le châssis moléculaire **1** ou support moléculaire est un décapeptide cyclique RAFT comprenant la séquence de dix résidus d'acides aminés : Glycine, proline, Lysine, Alanine, Lysine, Glycine, Proline, Lysine, Lysine, Lysine **-Gₐ**-**P_{b}-K_{c}-A_{d}-Kₑ-G_{f}-P_{g}-Kₕ-Kᵢ-Kⱼ.** Les enchaînements des résidus d'acides aminés : Glycine-Proline **-G_{a;f}** ; **P_{b;g}-** constituent des coudes **2** définissant un plan moyen **Pm**. Le châssis moléculaire **1** présente une première face supérieure **Fₛ** présentant quatre résidus d'acide aminé Lysine **K_{c}**, **Kₑ**, **Kₕ** et **Kⱼ** et une deuxième face inférieure **Fᵢ** présentant un seul résidu d'acide aminé Lysine **Kᵢ**. Le choix d'un décapeptide comme châssis moléculaire **1** permet la fixation de quatre molécules de ciblage **3**.

Les molécules de ciblage **3** sont des pentapeptides cycliques comprenant la séquence RGD spécifique de l'intégrine, et plus précisément, l'enchaînement : Arginine - Glycine - Acide aspartique - Phénylalanine - Lysine **-RGDfK-.** L'intégrine, et plus particulièrement l'intégrine αᵥβ₃, cible de la séquence pré-citée, est surexprimée par les zones néo-angiogéniques et par de nombreuses lignées de cellules tumorales humaines.

Les molécules de ciblage **3** sont couplées aux quatre résidus d'acides aminés Lysine **K_{c}**, **Kₑ**, **Kₕ** et **Kⱼ** de la face dite supérieure **Fₛ** du châssis moléculaire **1** via des liaisons oximes.

Le fluorophore **4** est le S0456 (marque déposée) ou le sel interne (sel trisodique) hydroxyde de 3,3-Dimethyl-2-[2-[2-chloro-3-[2-[1,3-dihydro-3,3-dimethyl-5-sulfo-1-(4-sulfobutyl)-2H-indol-2-ylidene]-ethylidene]-1-cyclohexen-1-yl]-ethenyl]-5-sulfo-1-(4-sulfobutyl)-3H-indolium. Le fluorophore S0456 **4** appartient à la famille des cyanines et fluoresce dans le domaine de longueurs d'onde compris entre 700 et 900 nm.

Le fluorophore S0456 est relié au châssis via un bras espaceur **8**, l'acide 5-(4-hydroxyphenyl)pentanoïque. Ce fluorophore présente une chaîne carbonée **4a** permettant la délocalisation des électrons. De plus ce fluorophore **4** S0456 présente des groupements sulfonates sur les groupements aromatiques **4b** conférant au traceur fluorescent **Tf2** une bonne solubilité en phase aqueuse.

Le bras espaceur **8** est fixé sur la face inférieure **Fᵢ** du châssis moléculaire 1 au niveau d'un résidu d'acide aminé Lysine **Kᵢ** via une liaison amide. Le bras espaceur **8** est fixé sur le fluorophore **4** au niveau du carbone à la place de l'élément chlore via une liaison éther et plus précisément via une liaison oxyphényl ce qui permet une délocalisation supplémentaire des électrons sur le groupement phényl. Par ailleurs, la liaison éther modifie peu la longueur d'onde du maximum d'émission du fluorophore **4**, la longueur d'onde du maximum d'émission étant diminuée de 10 à 15 nm par rapport au fluorophore **4** non fixé au châssis **1** via le bras espaceur **8**.

Le traceur selon l'invention diffère du traceur selon l'art connu en ce que le fluorophore est relié au châssis via un bras espaceur **8** reliant un carbone central de la chaîne carbonée à la lysine **Kᵢ** de la face inférieure **Fᵢ** du châssis ce qui permet l'emploi de fluorophore moins onéreux que ceux utilisés selon l'art antérieur.

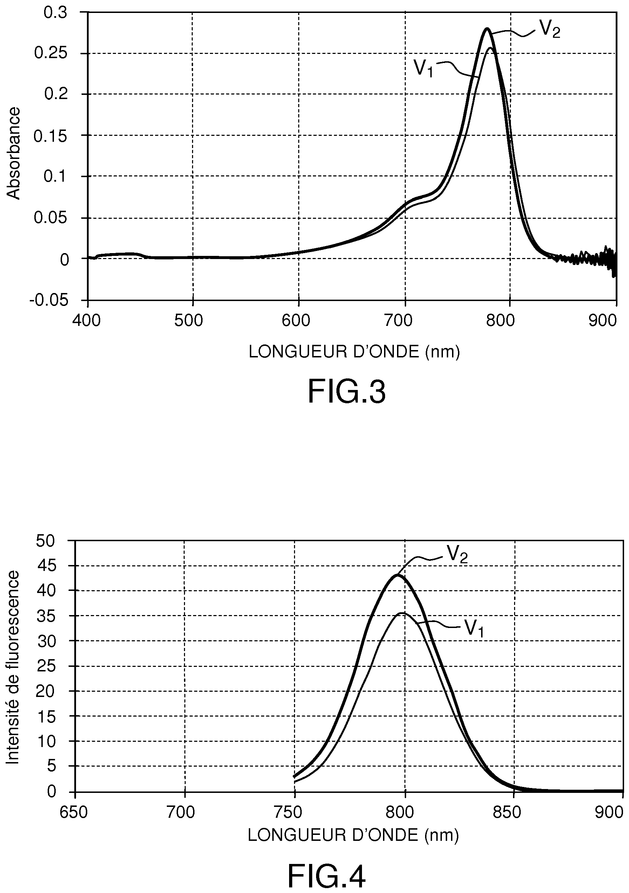

La figure 3 est une superposition du spectre d'absorption d'un traceur fluorescent selon l'art connu (courbe **V1**) et du traceur fluorescent selon l'invention (courbe **V2**). Les deux courbes présentent un pic d'absorption dont les valeurs maximales d'absorption apparaissent à une longueur d'onde de 781 nm pour un traceur **Tf1** selon l'art connu et de 778 nm pour le traceur **Tf2** selon l'invention.

Par ailleurs, la figure 4 est une superposition des spectres d'émission d'un traceur fluorescent **Tf1** selon l'art connu (courbe **V1**) et du traceur fluorescent **Tf2** selon l'invention (courbe **V2**). Les deux courbes présentent un pic dont les valeurs maximales d'émission apparaissent à une longueur d'onde de 801 nm pour un traceur **Tf1** selon l'art connu et de 797 nm pour le traceur **Tf2** selon l'invention.

Les figures 3 et 4 montrent bien que les propriétés optiques du traceur fluorescent selon l'art connu **Tf1** et selon l'invention **Tf2** sont sensiblement équivalentes. Aussi, le traceur **Tf2** selon l'invention semble parfaitement adapté au dispositif d'imagerie Fluobeam (marque déposée) de Fluoptics.

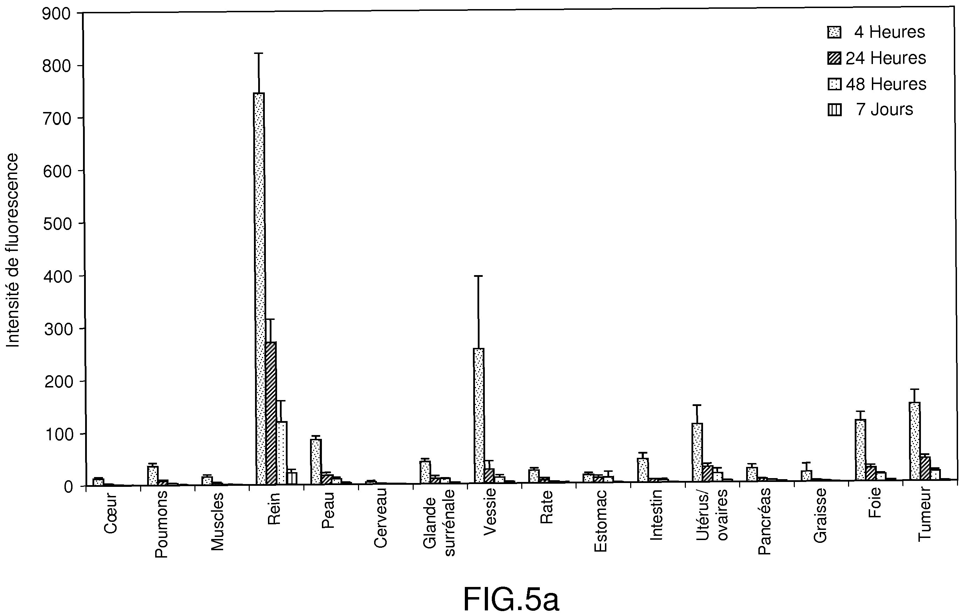

La figure 5a représente la distribution tissulaire d'un traceur **Tf1** selon l'art connu à différents temps post-injection.

En l'espèce, la fluorescence a été mesurée à des temps post-injection de 4h, 24h, 48h puis de sept jours, les organes ou tissus étudiés sont : le coeur, les poumons, les muscles, le rein, la peau, le cerveau, les glandes surrénales, la vessie, la rate, l'estomac, les intestins, les ovaires et l'utérus, le pancréas, les graisses, le foie et une tumeur mammaire murine sous-cutanée (Ts/Apc).

Le diagramme montre que l'intensité de fluorescence est la plus importante à un temps post-injection du traceur Tf1 de 4h et diminue après 24h. Après sept jours, l'intensité de fluorescence dans les organes et tissus est quasi nulle.

En outre, le diagramme montre que le traceur **Tf1** selon l'art connu est particulièrement bien approprié pour cibler une tumeur surexprimant l'intégrine αᵥβ₃. Par ailleurs, ce diagramme montre également une accumulation importante du traceur Tf1 dans les reins dès 4 heures post-injection, démontrant une élimination rapide du produit par voie rénale.

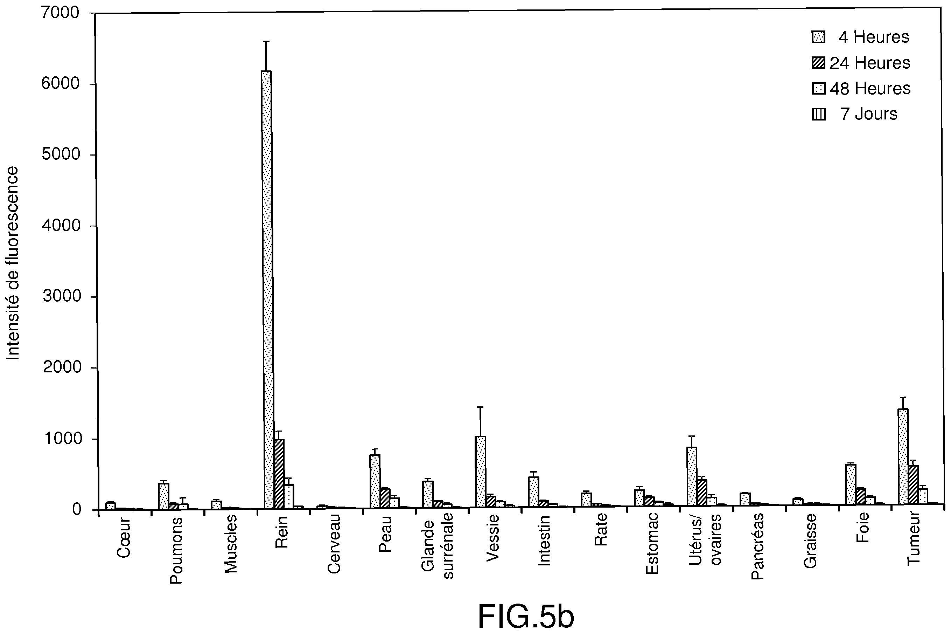

La figure 5b représente l'intensité de la fluorescence d'un traceur **Tf2** selon l'invention dans les mêmes organes et tissus que ceux étudiés figure 5a.

La distribution tissulaire du traceur **Tf2** selon l'invention est similaire à la distribution observée pour le traceur **Tf1** selon l'art connu.

Par ailleurs, l'affinité du traceur **Tf2** selon l'invention est similaire à l'affinité d'un traceur **Tf1** tel que le Cy5-RAFT-(c[RGDfK])₄ selon l'art connu. Ceci s'explique par le fait que les molécules de ciblage sont identiques et représentées en quantités identiques dans le traceur **Tf1** selon l'art connu et le traceur **Tf2** selon l'invention.

Par contre, le traceur **Tf2** selon l'invention présente une affinité bien plus élevée qu'un traceur monomérique présentant la molécule de ciblage représentée en un unique exemplaire.

Selon un autre aspect de l'invention, il est proposé un procédé de réalisation du traceur **Tf2** comprenant une étape de couplage du fluorophore **4** avec le châssis RAFT **1** de sorte à former un châssis RAFT fluorescent préalablement à l'étape de couplage des molécules de ciblage **3**.

Ce procédé de réalisation diminue les pertes de matières premières, et plus particulièrement celles des molécules de ciblage RGD et permet d'éviter l'étape d'activation du fluorophore avant l'étape de couplage.

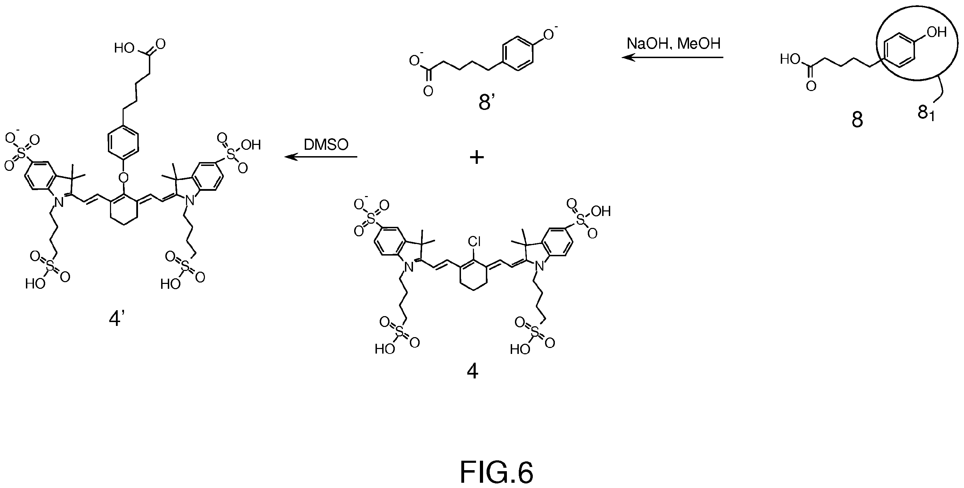

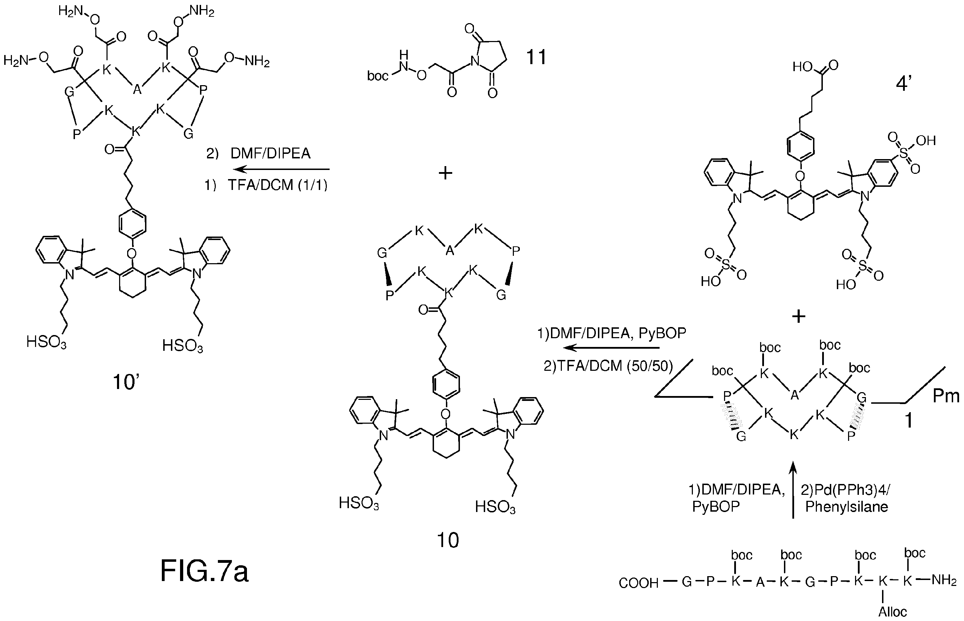

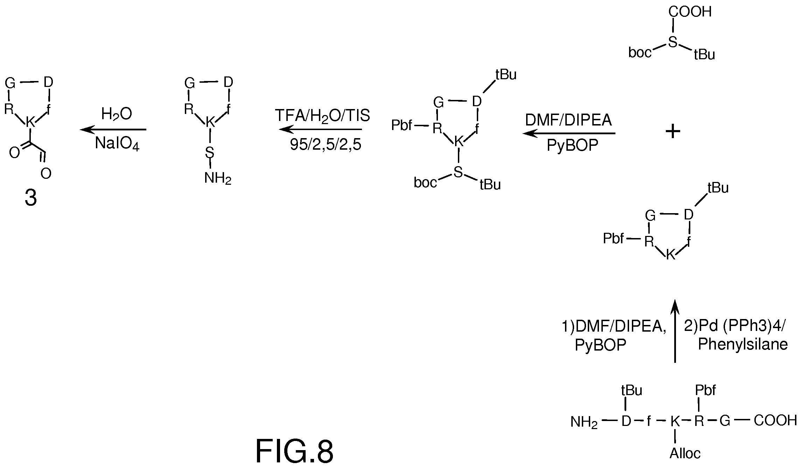

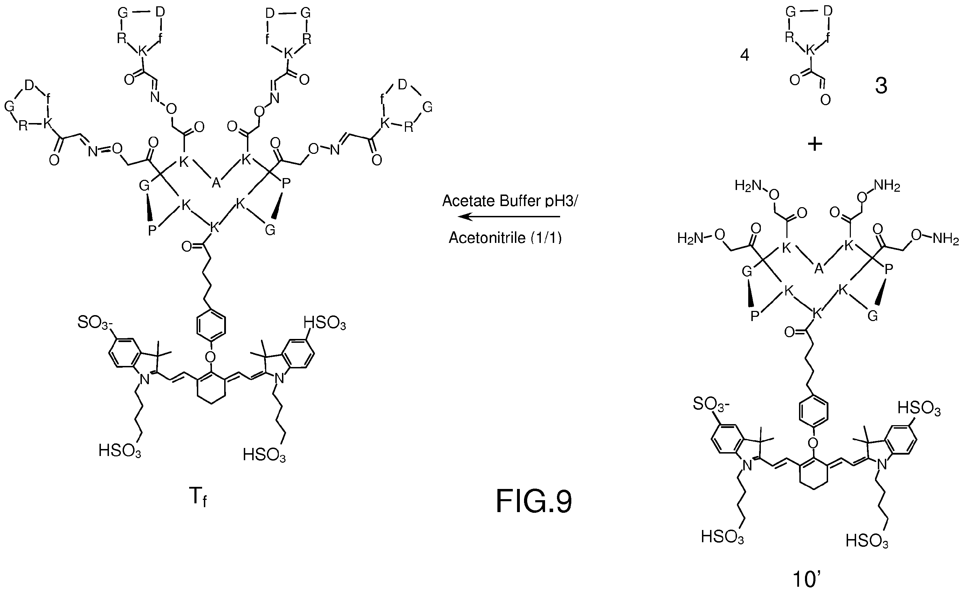

Le procédé comprend :
- une première étape, illustrée sur la figure 6, de préparation d'un fluorophore modifié **4'** dans laquelle le bras espaceur **8** est couplé au fluorophore **4**,
- une deuxième étape, représentée sur la figure 7a, dans laquelle le châssis RAFT **1** est synthétisé,
- une troisième étape, représentée sur la figure 7a, dans laquelle le châssis RAFT **1** est couplé au fluorophore modifié **4'** de sorte à former un châssis fluorescent **10**,
- une quatrième étape, illustrée sur la figure 7a, dans laquelle un précurseur **11** de liaison oxime est greffé au châssis RAFT **1** sur les résidus Lysine de la face dite supérieure **Fₛ** de sorte à former un châssis fluorescent modifié **10'**,
- une cinquième étape, représentée sur la figure 8, dans laquelle les molécules de ciblage **3** sont synthétisées, et
- une sixième étape, illustrée sur la figure 9, de couplage entre les molécules de ciblage **3** et le châssis fluorescent modifié **10'.**

Plus précisément, la figure 6 présente les étapes de préparation du fluorophore modifié **4'**, décrite notamment dans le document de Hyun, H. et al, «c-GMP-compatible preparative scale of near-infrared fluorophores. Contrast Media Mol. Imaging, 2012, 7:516 »*.* Le bras espaceur **8**, acide 5-(4-hydroxyphenyl)pentanoïque comprend un premier groupement terminal **8₈₁** phénol. Le phénol est convertit en phénolate, plus réactif que le phénol, dans une solution d'hydroxyde de sodium dans le méthanol pour former un bras espaceur modifié **8'.** Le bras espaceur **8'** obtenu est ensuite mélangé au fluorophore **4**, ici le S0456 (ou sel interne (sel trisodique) hydroxyde de 3,3-Dimethyl-2-[2-[2-chloro-3-[2-[1 ,3-dihydro-3,3-dimethyl-5-sulfo-1-(4-sulfobutyl)-2H-indol-2-ylidene]-ethylidene]-1-cyclohexen-1-yl]-ethenyl]-5-sulfo-1-(4-sulfobutyl)-3H-indolium) dans le DMSO acronyme de « DiMéthylSulfOxyde » pour obtenir le fluorophore modifié **4'** constitué du fluorophore 4 sur lequel est greffé le bras espaceur **8'.**

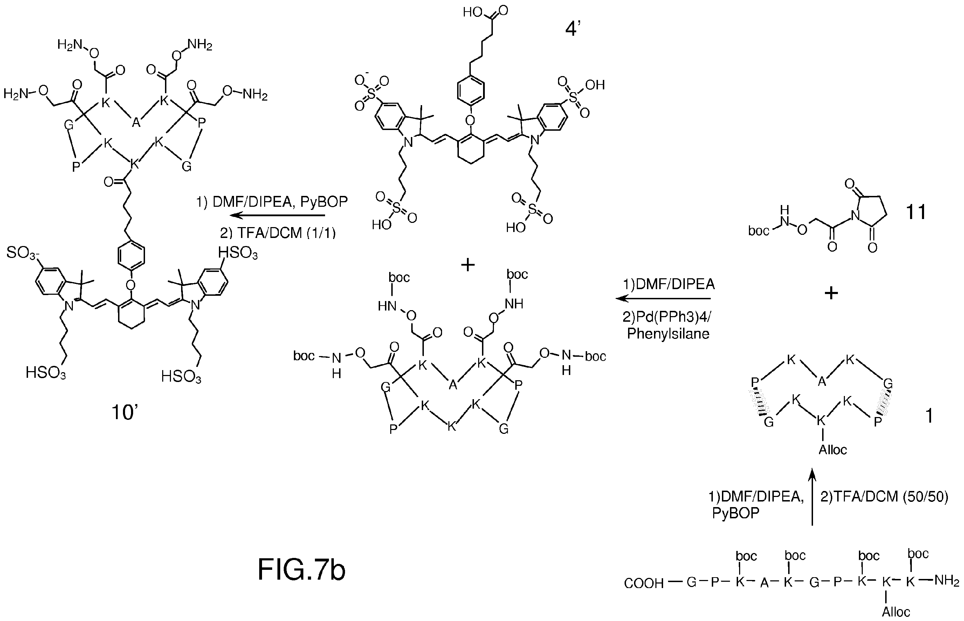

La figure 7a présente les deuxième, troisième et quatrième sous-étapes du procédé. Dans un premier temps, un décapeptide linaire comprenant la séquence de résidus d'acides aminés [-K(Boc)-K(Alloc)-K(Boc)-P-G-K(Boc)-A-K(Boc)-P-G-] est synthétisé sur résine, les groupements BOC et ALLOC étant des groupements protecteurs de sorte à permettre, par la suite, une fonctionnalisation régiosélective du châssis fluorescent. Après décrochage de la résine, le décapeptide est cyclisé et le résidu Lysine protégé par le groupement (Alloc) est déprotégé en présence de palladium (Pd⁰) et de phénylsilane de sorte à former un châssis RAFT **1** présentant un plan moyen **Pm.** Notons que la troisième étape et la quatrième étape peuvent être interverties comme illustré sur la figure 7b. En l'espèce, dans un premier temps, les groupements protecteurs BOC sur les résidus Lysine de la face dite supérieure **Fₛ** sont clivés en milieu acide puis des précurseurs d'oxyamine protégés **11** sont greffés sur les résidus Lysine via une liaison amide. Le résidu Lysine situé sur la face inférieure **Fᵢ** protégé par le groupement (Alloc) est déprotégé de sorte à permettre le greffage du fluorophore modifié 4' pour former un châssis fluorescent modifié 10'.

Le fluorophore modifiée **4'** est ensuite greffé sur le résidu Lysine déprotégé pour former un châssis fluorescent **10.** Les groupements protecteurs BOC sur les résidus Lysine de la face dite supérieure **Fₛ** sont clivés en milieu acide puis un précurseur d'oxyamine protégé **11** est greffé sur les résidus Lysine via une liaison oxime pour former le châssis RAFT fluorescent modifié **10'.**

Il est intéressant de noter que la sous-étape d'activation du fluorophore **4** indispensable dans le procédé selon l'état de la technique n'est pas nécessaire dans le procédé selon l'invention.

La figure 8 présente les étapes de synthèse de la molécule de ciblage **3.** Dans un premier temps, le pentapeptide linéaire comprenant la séquence de résidus d'acides aminés RGD spécifique des intégrines est synthétisé sur une résine. En l'espèce, le pentapeptide comprend la séquence [-D(tBu)-f-K(Alloc)-R(Pbf)-G-]. Après décrochage du peptide de la résine, celui-ci est cyclisé puis le résidu Lysine protégé par le groupement Alloc est ensuite déprotégé en présence de palladium (Pd⁰) et de phénylsilane. Un résidu sérine protégé est ensuite greffé sur le résidu Lysine avant déprotection totale du pentapeptide en milieu acide. La fonction alcool de la sérine est ensuite oxydé avec du périodate de sodium dans l'eau pour obtenir un groupement aldéhyde.

La figure 9 illustre l'étape de couplage entre le châssis fluorescent modifié **10'** et les pentapeptides cycliques **3**.

Dans le procédé revendiqué, le fluorophore commercial **4** intervient très tôt dans le processus de synthèse du traceur fluorescent **Tf2** par rapport au procédé de l'état de la technique.

Or, les exigences de pureté et de qualité relatives aux matières premières incorporées dans des formulations destinées à l'administration humaine intervenant tôt dans le procédé de synthèse sont moindres que pour les matières premières intervenant en fin de procédé comme cela est le cas dans le procédé de l'état de la technique. En l'espèce, le fluorophore **4** peut donc être de qualité et de pureté inférieures à celles requises lors de la synthèse selon le procédé de l'état de la technique, ce qui contribue de manière significative à la baisse du coût d'achat du fluorophore **4**.

De plus, contrairement au procédé décrit dans l'état de la technique, l'étape d'activation du fluorophore préalable à l'étape de couplage entre le fluorophore modifié **10'** et le châssis RAFT **1** n'est pas nécessaire ce qui permet de diminuer encore les coûts de fabrication du traceur fluorescent **Tf2** selon l'invention.

Aussi, la synthèse de 15 g de traceur fluorescent **Tf2** requiert 30g de RAFT, 105g de RGD et 13g de fluorophore.

Le traceur fluorescent **Tf2** proposé dans la présente invention est adapté pour une utilisation avec un dispositif d'imagerie de fluorescence de type Fluobeam (marque déposée), il permet de diminuer de manière importante les coûts d'une part en permettant l'utilisation de fluorophore moins onéreux mais aussi en améliorant le procédé de fabrication permettant ainsi de supprimer des étapes réactionnelles jusqu'alors essentielles dans le procédé proposé selon l'état de la technique.

## Revendications

1. Traceur fluorescent **(Tf2)** comprenant :
- un décapeptide cyclique **(1)** constitué de l'enchaînement des dix résidus d'acides aminés : -Glycine-Proline-Lysine-Alanine-Lysine-Glycine-Proline-Lysine-Lysine-Lysine **(-Gₐ-P_{b}-K_{c}-A_{d}-Kₑ-G_{f}-P_{g}-Kₕ-Kᵢ-Kⱼ-)** configuré de sorte à définir un plan moyen **(Pm)** définissant une première face supérieure **(Fₛ)** présentant quatre résidus d'acides aminés Lysine **(K_{c}, Kₑ**, **Kₕ**, **Kⱼ)** et une deuxième face inférieure **(Fᵢ)** présentant un résidu d'acide aminé Lysine **(Kᵢ)**,
- quatre molécules de ciblage identiques **(3)**, les molécules de ciblage étant des pentapeptides cycliques constitués de l'enchaînement de résidus d'acides aminés : Arginine - Glycine - Acide aspartique - Phénylalanine - Lysine **(RGDKf)**, chacune des molécules de ciblage **(3)** étant fixée sur un résidu d'acide aminé Lysine **(K_{c}, Kₑ**, **Kₕ**, **Kⱼ)** différent de la première face supérieure **(Fₛ)** via une liaison oxime, et
- un fluorophore **(4)** sel interne (sel trisodique) hydroxyde de 3,3-Dimethyl-2-[2-[2-chloro-3-[2-[1,3-dihydro-3,3-dimethyl-5-sulfo-1-(4-sulfobutyl)-2H-indol-2-ylidene]-ethylidene]-1-cyclohexen-1-yl]-ethenyl]-5-sulfo-1-(4-sulfobutyl)-3H-indolium, présentant un enchaînement de doubles liaisons avec un carbone central, le fluorophore S0456 **(4)** étant fixé sur la deuxième face inférieure **(Fᵢ)** du plan moyen **(Pm)** via un bras espaceur **(8)** reliant le carbone central de l'enchaînement de doubles liaisons du fluorophore **(4)**, via une liaison éther, et, le résidu d'acide aminé lysine **(Kᵢ)** de la face inférieure **(Fᵢ)** du décapeptide **(1)** via une liaison amide, le bras espaceur étant l'acide 5-(4-hydroxyphenyl)pentanoïque.

2. Procédé de synthèse d'un traceur fluorescent, le traceur fluorescent **(Tf2)** comprenant :
- un décapeptide cyclique **(1)** constitué de l'enchaînement des dix résidus d'acides aminés : -Glycine-Proline-Lysine-Alanine-Lysine-Glycine-Proline-Lysine-Lysine-Lysine- **(-Gₐ-P_{b}-K_{c}-A_{d}-Kₑ-G_{f}-P_{g}-Kₕ-Kᵢ-Kⱼ-)** configuré de sorte à définir un plan moyen **(Pm)** définissant une première face supérieure **(Fₛ)** présentant quatre résidus d'acide aminé Lysine **(K_{c}, Kₑ**, **Kₕ**, **Kⱼ)** et une deuxième face inférieure **(Fᵢ)** présentant un résidu d'acide aminé Lysine **(Kᵢ),**
- quatre molécules de ciblage identiques **(3)**, les molécules de ciblage étant des pentapeptides cycliques constitués de l'enchaînement de résidus d'acides aminés : Arginine - Glycine - Acide aspartique - Phénylalanine - Lysine **(-RGDfK-),** chacune des molécules de ciblage **(3)** étant fixée sur un résidu d'acide aminé Lysine différent de la première face supérieure **(Fₛ)** via une liaison oxime, et
- un fluorophore **(4)** sel interne (sel trisodique) hydroxyde de 3,3-Dimethyl-2-[2-[2-chloro-3-[2-[1,3-dihydro-3,3-dimethyl-5-sulfo-1-(4-sulfobutyl)-2H-indol-2-ylidene]-ethylidene]-1-cyclohexen-1-yl]-ethenyl]-5-sulfo-1-(4-sulfobutyl)-3H-indolium, présentant un enchaînement de doubles liaisons avec un carbone central, le fluorophore **(4)** étant fixé sur la deuxième face inférieure **(Fᵢ)** du plan moyen **(Pm)** via un bras espaceur **(8)** reliant le carbone central de l'enchaînement de doubles liaisons du fluorophore via une liaison éther et le résidu d'acide aminé lysine **(Kᵢ)** du décapeptide **(1)** via une liaison amide, le bras espaceur étant un acide 5-(4-hydroxyphenyl)pentanoïque,
le procédé comprenant une étape de fixation du fluorophore **(4)** sur le décapeptide **(1)** via le bras espaceur **(8)** préalablement à une étape de fixation des molécules de ciblage **(3)**.

## Patentansprüche

1. Fluoreszierender Tracer (Tf2), Folgendes beinhaltend:
- ein zyklisches Dekapeptid (1), gebildet aus der Verkettung der zehn Aminosäurerückstände: -Glycin-Prolin-Lysin-Alanin-Lysin-Glycin-Prolin-Lysin-Lysin-Lysin (-Gₐ-P_{b}-K_{c}-A_{d}-Kₑ-G_{f}-P_{g}-Kₕ-Kᵢ-Kⱼ-), dergestalt konfiguriert, dass sie eine mittlere Ebene (Pm) definiert, welche eine erste obere Seite (Fₛ) definiert, welche vier Lysin-Aminosäurerückstände (K_{c}, Kₑ, Kₕ, Kⱼ) aufweist und eine zweite, untere Seite (Fᵢ) definiert, welche einen Lysin-Aminosäurerückstand (Kᵢ) aufweist,
- vier identische Targeting-Moleküle (3), wobei die Targeting-Moleküle zyklische Pentapeptide sind, welche aus der Verkettung von Aminosäurerückständen gebildet werden: Arginin - Glycin - Aspartamsäure - Phenylalanin - Lysin (RGDKf), wobei jedes der Targeting-Moleküle (3) an einen unterschiedlichen Lysin-Aminosäurerückstand (K_{c}, Kₑ, Kₕ, Kⱼ) der ersten oberen Seite (Fₛ) über eine Oximverbindung gbunden ist, und
- ein Fluorophor (4), ein inneres Salz (Trinatriumsalz) vom Typ 3,3-Dimethyl-2-[2-[2-chlor-3-[2-[1-[1,3-dihydro-3,3-dimethyl-5-sulfo-1-(4-sulfobutyl)-2H-indol-2-yliden]-ethyliden]-1-cyclohexen-1-yl]-ethenyl]-5-sulfo-1-(4-sulfobutyl)-3H-indolium-Hydroxid, welches eine Verkettung von Doppelverbindungen mit einem zentralen Kohlenstoffatom aufweist, wobei das Fluorophor S0456 (4) an die zweite untere Seite (Fᵢ) der mittleren Ebene (Pm) über einen Abstandsarm (8) gebunden ist, welcher das zentrale Kohlenstoffatom der Verkettung von Doppelverbindungen des Fluorophors (4) über eine Etherverbindung verbindet, und der Lysin-Aminosäurerückstand (Kᵢ) der unteren Seite (Fᵢ) des Dekapeptids (1) über eine Amidverbindung verbindet, wobei der Abstandsarm aus 5-(4-Hydroxyphenyl)-Pentansäure besteht.

2. Verfahren zur Synthese eines fluoreszierenden Tracers, wobei der fluoreszierende Tracer (Tf2) Folgendes beinhaltet:
- ein zyklisches Dekapeptid (1), gebildet aus der Verkettung der zehn Aminosäurerückstände: -Glycin-Prolin-Lysin-Alanin-Lysin-Glycin-Prolin-Lysin-Lysin-Lysin- (-Gₐ-P_{b}-K_{c}-A_{d}-Kₑ-GᵣP_{g}-Kₕ-Kᵢ-Kⱼ-), dergestalt konfiguriert, dass sie eine mittlere Ebene (Pm) definiert, welche eine erste obere Seite (Fₛ) definiert, welche vier Lysin-Aminosäurerückstände (K_{c}, Kₑ, Kₕ, Kⱼ) aufweist und eine zweite, untere Seite (Fᵢ) definiert, welche einen Lysin-Aminosäurerückstand (Kᵢ) aufweist,
- vier identische Targeting-Moleküle (3), wobei die Targeting-Moleküle zyklische Pentapeptide sind, welche aus der Verkettung von Aminosäurerückständen gebildet werden: Arginin - Glycin - Aspartamsäure - Phenylalanin - Lysin (-RGDKf-), wobei jedes der Targeting-Moleküle (3) an einen unterschiedlichen Lysin-Aminosäurerückstand der ersten oberen Seite (Fₛ) über eine Oximverbindung gbunden ist, und
- ein Fluorophor (4), ein inneres Salz (Trinatriumsalz) vom Typ 3,3-Dimethyl-2-[2-[2-chlor-3-[2-[1-[1,3-dihydro-3,3-dimethyl-5-sulfo-1-(4-sulfobutyl)-2H-indol-2-yliden]-ethyliden]-1-cyclohexen-1-yl]-ethenyl]-5-sulfo-1-(4-sulfobutyl)-3H-indolium-Hydroxid, welches eine Verkettung von Doppelverbindungen mit einem zentralen Kohlenstoffatom aufweist, wobei das Fluorophor (4) an die zweite untere Seite (Fᵢ) der mittleren Ebene (Pm) über einen Abstandsarm (8) gebunden ist, welcher das zentrale Kohlenstoffatom der Verkettung von Doppelverbindungen des Fluorophors über eine Etherverbindung verbindet, und der Lysin-Aminosäurerückstand (Kᵢ) des Dekapeptids (1) über eine Amidverbindung verbindet, wobei der Abstandsarm aus 5-(4-Hydroxyphenyl)-Pentansäure besteht,
wobei das Verfahren einen Schritt des Bindens des Fluorophors (4) am Dekapeptid (1) über den Abstandsarm (8) vor dem Schritt des Bindens der Targeting-Moleküle (3) beinhaltet.

## Claims

1. A fluorescent tracer (Tf2), comprising:
- a cyclic decapeptide (1) consisting of the sequence of the ten amino acid residues: -Glycine-Proline-Lysine-Alanine-Lysine-Glycine-Proline-Lysine-Lysine-Lysine (-Gₐ-P_{b}-K_{c}-A_{d}-Kₑ-G_{f}-P_{g}-Kₕ-Kᵢ-Kⱼ-) configured so as to define a mean plane (Pm) defining a first upper face (Fₛ) having four lysine amino acid residues (K_{c}, Kₑ, Kₕ, Kⱼ) and a second lower face (Fᵢ) having one lysine amino acid residue (Kᵢ),
- four identical targeting molecules (3), the targeting molecules being cyclic pentapeptides consisting of the sequence of amino acid residues: Arginine - Glycine - Aspartic acid - Phenylalanine - Lysine (RGDKf), each of the targeting molecules (3) being fixed to a different lysine amino acid residue (K_{c}, Kₑ, Kₕ, Kⱼ) of the first upper face (Fₛ) via an oxime bond, and
- a fluorophore (4), 3,3-Dimethyl-2-[2-[2-chloro-3-[2-[1,3-dihydro-3,3-dimethyl-5-sulfo-1-(4-sulfobutyl)-2H-indol-2-ylidene]ethylidene]-1-cyclohexen-1-yl]ethenyl]-5-sulfo-1-(4-sulfobutyl)-3H-indolium hydroxide, inner salt (trisodium salt), having a sequence of double bonds with a central carbon, the fluorophore S0456 (4) being fixed to the second lower face (Fᵢ) of the mean plane (Pm) via a spacer arm (8) connecting the central carbon of the sequence of double bonds of the fluorophore (4) via an ether bond, and the lysine amino acid residue (Kᵢ) of the lower face (Fᵢ) of the decapeptide (1) via an amide bond, the spacer arm being 5-(4-hydroxyphenyl)pentanoic acid.

2. A process for synthesizing a fluorescent tracer, the fluorescent tracer (Tf2) comprising:
- a cyclic decapeptide (1) consisting of the sequence of the ten amino acid residues: -Glycine-Proline-Lysine-Alanine-Lysine-Glycine-Proline-Lysine-Lysine-Lysine (-Gₐ-P_{b}-K_{c}-A_{d}-Kₑ-G_{f}-P_{g}-Kₕ-Kᵢ-Kⱼ-) configured so as to define a mean plane (Pm) defining a first upper face (Fₛ) having four lysine amino acid residues (K_{c}, Kₑ, Kₕ, Kⱼ) and a second lower face (Fᵢ) having one lysine amino acid residue (Kᵢ),
- four identical targeting molecules (3), the targeting molecules being cyclic pentapeptides consisting of the sequence of amino acid residues: Arginine - Glycine - Aspartic acid - Phenylalanine - Lysine (-RGDfK-), each of the targeting molecules (3) being fixed to a different lysine amino acid residue of the first upper face (Fₛ) via an oxime bond, and
- a fluorophore (4), 3,3-Dimethyl-2-[2-[2-chloro-3-[2-[1,3-dihydro-3,3-dimethyl-5-sulfo-1-(4-sulfobutyl)-2H-indol-2-ylidene]ethylidene]-1-cyclohexen-1-yl]ethenyl]-5-sulfo-1-(4-sulfobutyl)-3H-indolium hydroxide, inner salt (trisodium salt), having a sequence of double bonds with a central carbon, the fluorophore (4) being fixed to the second lower face (Fᵢ) of the mean plane (Pm) via a spacer arm (8) connecting the central carbon of the sequence of double bonds of the fluorophore via an ether bond, and the lysine amino acid residue (Kᵢ) of the decapeptide (1) via an amide bond, the spacer arm being 5-(4-hydroxyphenyl)pentanoic acid,
the process comprising a step of fixing the fluorophore (4) to the decapeptide (1) via the spacer arm (8) prior to a step of fixing the targeting molecules (3).
